Europäisches Patentamt

**European Patent Office**

**Office européen des brevets**

(11) Publication number: **0 045 614**

**A1**

(12) # EUROPEAN PATENT APPLICATION

(21) Application number: **81303445.1**

(22) Date of filing: **27.07.81**

(51) Int. Cl.³: **C 02 F 3/28**
**C 02 F 11/04, A 01 C 3/00**
**A 01 C 3/02, C 12 P 5/00**
**C 12 P 5/02**

(30) Priority: **31.07.80 NZ 193588**

(43) Date of publication of application:
**10.02.82 Bulletin 82/6**

(84) Designated Contracting States:
**AT BE CH DE FR GB IT LI LU NL SE**

(71) Applicant: **Romero-Sierra, Cesar**
**P.O. Box 32 Graham Manor**
**Bath Ontario(CA)**

(72) Inventor: **Romero-Sierra, Cesar**
**P.O. Box 32 Graham Manor**
**Bath Ontario(CA)**

(74) Representative: **Skailes, Humphrey John et al,**
**Frank B. Dehn & Co. Imperial House 15-19 Kingsway**
**London WC2B 6UZ(GB)**

(54) **Biodegradation of carbonaceous materials.**

(57) A process for converting biodegradable carbon-containing materials into substances containing a significant proportion of methylene groups, which process comprises subjecting the biodegradable carbon-containing materials to bacterial action in a container under conditions of temperature suitable for the bacterial flora present to thrive, of pressure such that any evolved gasses are retained in contact with the carbon-containing materials, of volume such that the free space in the container is minimised, and in the presence of at least sufficient water to maintain the bacterial flora alive.

EP 0 045 614 A1

0045614

## BIODEGRADATION OF CARBONACEOUS MATERIALS

This invention is concerned with biochemical processes whereby carbonaceous materials of a wide variety may be converted relatively cheaply into a material approximating more or less closely to crude oil as currently obtained from oil wells.

Alternatively, this invention is concerned with biochemical processes whereby carbonaceous materials of a wide variety may be converted relatively cheaply into a material containing chains of methylene groups, and which can therefore readily be treated by known technology to provide the types of materials, including oils and gasolines, currently obtained from crude oil.

During the last century or so that crude oil has been recovered, refined, used and wasted, in addition to very considerable research effort directed toward techniques to deplete the world's resources of oil more rapidly and comprehensively, a relatively small amount of research has been directed toward discovering where the oil came from in the first place, and particularly how it came to be formed. That crude oil is most likely to be of a biological nature is clearly indicated by the presence in it of many chemical compounds closely related to living systems; for example both pigments and lignin have been identified in crude oils.

It is also well known that many living biological systems are quite capable of producing materials that are essentially higher molecular weight hydrocarbons. Some of these in fact have been used as lubricants, as fuels, and for lighting purposes: for example, animal-based greases, whale oil, the many vegetable oils, and higher polymeric systems such as rubber and resins. Thus it is clearly apparent that given the right conditions, living biological systems are eminently capable of converting an organic substrate into a relatively high molecular weight hydrocarbon material.

The major, and frequently determining, problems facing these biological processes are two: they are slow, and relatively uncontrollable. For example, in producing corn oil generally only one corn harvest per annum is possible, and that harvest is subject to climatic vagaries as to both quantity and quality. Similarly natural rubber obtained by tapping annually trees of the hevea brasiliensis family is in direct competition with a variety of synthetic materials derived, in the end, from crude oil.

Notwithstanding these problems, in one particular area of biological chemical processing man has learnt how to overcome many of these difficulties. A level of control can be achieved which permits the economic and industrial exploitation of these biological processes. This area is the field of microbiology. If the preparation of such products as beer, wine, yoghurt, bread and the like are taken into account, microbiological processes are probably the oldest commerically practised chemical processes. However the deliberate exploitation of bacteria in the service of man is only about a century old.

During the last hundred years or so bacteria of many varieties, including mutant strains produced by the intervention of man into the genetic processes, have been isolated and cultured that are capable of many specific tasks. Indeed a large segment of the modern drug industry relies almost totally on some form of bacteria to produce if not the required drug, then a lineal precursor of it. Thus bacteria are now identified which will process a given nutrient system to produce quite complex molecules of relatively high molecular weights. Similarly bacteria are also now identified which will more, or less, efficiently degrade many materials into substances of low molecular weight. Frequently these low molecular weight materials are exceedingly simple molecules, such as water, acetic acid, carbon dioxide, methane, ammonia, hydrogen sulphide, and so forth.

It is thus clear that microbiological processes can both process comparatively complex organic materials and also convert them into a wide range of other, quite different, organic materials which need not be relatively simple molecules.

But from all of this available data, varying from the processing of sewage and manure to recover some methane (which is generally simply profligately wasted by a sewage plant through combustion in a flare-stack) to the processing of complex broths to make antibiotics, one very significant feature is totally missing. No real attempt has been made, or apparently even contemplated, to persuade bacteria to process organic materials into a product resembling, more or less closely, crude oil. In view of the fact that living systems other than bacteria are known to be capable of producing relatively long chain hydrocarbons this is surprising. It is especially surprising in view of the fact that bacteria are known to produce methane, the simplest of all hydrocarbons.

It has now been discovered that bacteria can be used in fact to process many forms of organic matter, particularly waste materials such as garbage and especially sewage solids, to a largely hydrocarbonaceous material having a resemblance to crude oil. The resemblance appears to be close enough to permit processing of this hydrocarbonaceous material by current oil refinery techniques to provide, particularly, gasolines and related light distillates.

Thus in its broadest aspect this invention provides a process for converting organic materials into a largely hydrocarbonaceous material resembling crude oil, which process comprises bacterially degrading the organic materials under conditions whereby the bacteria produce significant quantities of the largely hydrocarbonaceous material.

Thus this invention provides a process for converting biodegradable carbon containing materials into substances containing a significant proportion of methylene groups, which process comprises subjecting the biodegradable carbon containing materials to bacterial action in a container under conditions of temperature suitable for the bacterial flora present to thrive, of pressure such that any evolved gasses are retained in contact with the carbon-containing materials, of volume such that the free space in the container is minimised, and in the presence of at least sufficient water to maintain the bacterial flora alive.

The major process parameters which provide the required conditions are as follows.

For many microbiological processes, specific bacteria are required, for example in the antibiotics industry and even in the brewing industry. However few organic substrates of the type being processed here, which are discussed in more detail below, in fact are truly sterile and totally free of bacteria. This is especially true of organic materials in which natural decay or rotting is already going on, such as sewage sludge. Generally speaking although it has not been found necessary to supplement this natural bacterial population, it has been found necessary to ensure that the size of this population is adequate for the process to proceed. This can be achieved either by adding a bacterial culture to a material relatively low in bacteria, or by allowing sufficient time for the indigenous bacterial flora to proliferate to a suitable level. Thus manure may need to be stored for a period of up to 15 days (depending on the storage conditions) to ensure that it contains an adequate bacterial population. Alternatively, for some particular possible feed materials it may be found necessary to use particular bacteria which will attack and degrade that substance. For example, in processing wood chips from lumber debris, material from rotting fallen trees could be used as the microbe source.

As a further possibility here the use of man-made bacteria can be considered. There seems to be no reason at all why the hydrocarbon producing propensities of a given strain, or flora, of bacteria which match a given carbonaceous substrate could not be optimised by the careful use of genetic engineering.

It is also possible to incorporate into the material to be processed additives which will promote bacterial activity, either as a bacterial food, or as a bacterial stimulant. For example, the addition of glycerin or sugar as a food, or of enzymes as stimulants, can be made. But care is needed in making such additions, as otherwise the characteristics of the bacterial flora may be changed, due to the development of particular strains in the flora at the expense of others. Thus an additive favouring the development of bacteria which produce alcohol, methane, or propane should not be used, or only used very cautiously.

It is desirable that oxygen is not present in the reactor to any great extent. From this it follows that anaerobic or facultative bacteria are required. The reason for this simply is to avoid reactions leading to the formation of oxygen-containing materials.

This low oxygen requirement does not preclude the presence of oxygen in related processes. Thus the pre-treatment of organic raw materials can be carried out under aerobic conditions, for example to provide an adequate bacterial flora in a given substrate material.

In most microbiological processes a considerable amount of water is generally present. For the purposes of this invention, water should be absent as far as is practicable. This is not to say that the system should be anhydrous, for the simple reason that bacteria cannot live in an anhydrous environment. Water is only essential in microbiological processes insofar as it is involved in the cell chemical processes, and is present in the cell membrane boundary. Thus the condition desirable for this invention is that the water content should ideally be only that amount needed to keep the bacteria alive and active. This ideal is almost impossible to obtain; the practical condition is to obtain a free water level that is as low as can be reasonably achieved.

It has been found, though, that the removal of water from natural biological fluids, especially urines, is not apparently necessary. It is also not necessary to limit the amount of water used in related processes, for example in separation techniques to recover products.

Bacteria will attack and degrade, in time, all natural materials and many of the synthetic materials present in wastes. All or any of these can thus be treated by this process.

Thus this process is peculiarly able to accept and process almost any of the materials currently regarded as garbage, and arising from both normal households and from many industries. A particularly attractive feed material at the present time is sewage sludge: it is available in large quantities, is replete with bacteria, and has to be treated in some way to render it innocuous anyway. Similar arguments apply to manure and faeces of all types, which, as a consequence of intensive farming systems, are becoming a disposal problem. But this is not to say that manure and the like are the only feedstocks: in some locations even now other organic materials may be an embarrassment thus making their processing desirable.

An important feature of the feed material is its available surface area. The ratio of surface to volume is important since it is surface contact that is essential for quick degradation. Thus one would not attempt to process scrap lumber: it is preferable to convert it to chips or flakes first. An alternative approach is to provide the bacterial flora with a relatively inert high surface area carrier. The presence of this carrier does not affect the biological processes in so far as their chemistry is concerned.

The carrier does however influence the product yields, or carbon conversions. It seems that such a carrier provides a means whereby the apparent viscosity of the mixture can be controlled thus facilitating the access of the bacteria to the substrate. Generally speaking for reasons which are not at all clear, a silica-containing material performs this function well. Thus silica sand, sodium silicate, potassium silicate (waterglass), various organic silicones, and various alumino silicates including Fuller's earth, bentonite, kaolin, and keiselguhr have been found to be effective.

As is normal for microbiological processes a nutrient medium is needed. In this process, however, the organic material generally provides sufficient nutrients.

Essentially, the medium has to be one which will serve to:

(a) maintain the growth of the bacteria;

(b) maintain as high a metabolic rate as possible;

and (c) facilitate surface contact between bacteria and substrate.

It does not follow that an aqueous medium is to be used, since minimal water is desired in the system. A hydrocarbon one could equally well be used.

The reaction conditions are also of importance. The four essential parameters are temperature, pressure, reactor content, and reactor content agitation.

As regards temperature, in many cases the system will be found to be self regulating, in the mesophilic range (that is, from $20^{\circ}C$ to $38^{\circ}C$ approximately). An ideal temperature is about $35^{\circ}C$. Whilst both cooler (i.e. psycrophilic) or hotter (i.e. thermophilic) procedures can be used, they are not recommended. The cooler processes generally are found to be too slow. The hotter processes generally are found to be too difficult to control as only a slight degree of overheating causes the death of the bacteria, but in appropriate cases, a faster fermentation can be obtained. Temperature control is effected by conventional means.

One rather peculiar temperature effect has been noted. As is well known, even a thermophilic bacterial flora has an upper temperature limit, above which death of the bacteria results. This proposition is the basis of the commonly used heat sterilization procedure for killing bacteria. Sterilization is generally carried out using steam, at a temperature in the vicinity of $100^{o}C$, or perhaps a little higher. In at least one instance, it has been found that high temperatures appear not to impair this process. A sample which had been heated to $150^{o}C$ apparently was not sterilized, as the reaction restarted, spontaneously, after the sample had cooled.

As regards pressure, it is necessary that all low molecular weight hydrocarbons be retained: thus in contrast to present procedures methane, etc. are not vented. Hence a reactor capable of withstanding some considerable pressure, possibly up to 1,000 ats, may be required. Bacteria will function adequately at least up to this order of pressure. The process is thus essentially a batch wise sealed reactor system.

In general, it is found that the higher the pressure, the slower microbiological processes become. In this process the possible influences of higher pressures can be overcome by providing a constant pressure, variable volume, reactor. The gasses released in the main reactor can be collected and stored in a secondary vessel. These stored gasses can then be returned to the main reactor, or even "fed" to another bacterial culture.

As regards reactor content, the major point is that an anaerobic process is being used. It is therefore desirable to minimise as far as possible the free space in the reactor, preferably by filling it with organic material for processing.

As regards agitation, it has been found not necessarily desirable deliberately to stir or agitate the reactor contents. It appears that it is better to leave the bacteria to float about under natural circumstances. Thus the proper choice of medium will generally obviate the need for agitation.

At this point in the process there are many ways in which the crude reactor product can be processed. The manner of treatment will largely depend upon the product desired. The material in the reactor could be used without further processing, for example as an oil-food, or oil-food additive. In that case, the proteinaceous material remaining from the bacterial cells is of some importance. Alternatively, the crude reactor product can be treated to provide a hydrocarbonaceous base material. In the reactor product, the hydrocarbonaceous material is bound up in the cells, in a manner analogous to fat cells in animals. The hydrocarbonaceous material is then recovered by breaking up these cells, in any convenient way. The recovered hydrocarbonaceous material can then be processed by conventional means to provide the desired products.

In order to free the oily material, it has been found advantageous to break up the biodegradable material and the bacterial flora in the reactor. This is conveniently achieved by pressurizing the reactor, ideally by decreasing its volume by means of a piston, to at least 400 psig. and by heating the reactor to at least $150^{\circ}$C, and preferably to at least $250^{\circ}$C. However reactor construction materials, especially seals, place some restraints on the temperatures which may be used.

Examples

In the following Examples a percentage conversion figure is given. This figure is an approximate calculation. It represents the percentage of the carbon in the original material subjected to bacterial action which is converted into hydrocarbonaceous material containing methylene groups ($-CH_2-$). Thus the analysis used was to determine the proportion of methylene groups in a sample by instrumental analysis techniques, and then relate that figure to the amount of carbon in the original material. The conversion figures given are approximate calculations since no precise carbon contents were measured for the feed materials taken. Representative carbon contents were calculated based on published analyses for the sorts of materials being handled. The enzyme mixture was a conventional compost heap starter mix.

Example I

The following components were mixed in a conventional household blender:

0.5 litres of stored human urine

0.5 kg fresh pig manure solid

125 gm Fuller's earth

30 ml sodium silicate

0.5 litre water

14 gm enzyme mixture

Of this mix, 800 ml was put into a 800 ml, pressure cylinder, and left at ambient temperature ($16^{\circ}C$ to $21^{\circ}C$) for three days. On the fourth day it was heated to $30^{\circ}C$ for 24 hours, and on the fifth heated to $150^{\circ}C$ for 2½ hours. During this last heating some leakage occurred past the cylinder seals. The released material was a greasy, sticky, dark brown liquid. After cooling, the cylinder was opened and a sample taken for analysis. The product remaining in the jar was a thick paste.  The calculated carbon conversion was 20.5%, ignoring the material lost in the last heating step.

Example 2

The procedure of Example 1 was repeated, the only change being to increase the water content to 1.0 litres.  In this case no leakage occurred in the final heating step.  After cooling, the cylinder was found to contain a brownish liquid under pressure. Analysis of a sample indicated a conversion of 11.3%.

Example 3

The following components were mixed in a conventional household blender:

0.5 litre of stored human urine

0.5 kg fresh solid pig manure

125 gm. Fuller's earth

30 ml sodium silicate

1 litre water

14 gm enzymes mixture

40 ml glycerine

Of this mix, 800 ml was then subjected to the same procedure as in Example 1.  No leakage occurred from the cylinder, which on cooling was found to contain a mixture of some solids and a brown liquid, under pressure.  Analysis of a sample indicated a conversion of 19%.

Example 4

The following components were mixed in a conventional household blender:

0.5 litre stored human urine

0.5 kg fresh solid pig manure

125 gm Fuller's earth

30 ml sodium silicate

1 litre water

14 gm enzymes mixture

40 ml glycerine

Of this mix, 800 ml was placed in a pressure vessel of 800 ml capacity. Four hours later, the cylinder was heated to 150°C for 2½ hours, and then left to stand at ambient temperature for three days. Although no leakage apparently occurred, no pressure was found when the cylinder was opened. Analysis of the product indicated a conversion of 5%.

Example 5

The following components were mixed in a conventional household blender:

250 ml urine (human)

500 ml water

250 gm liquid pig manure

125 gm Fuller's earth

Of this mixture, 800 ml was placed in an 800 ml cylinder. 24 hours later the cylinder was heated to 150°C for 2½ hours and then left to stand for 12 hours. No leakage was observed. Sample analysis indicated a conversion of 5%.

- 14 -

0045614

Example 6

In this case, Example 5 was repeated, with the addition of 30 ml glycerine to the initial mixture. Sample analysis indicated a conversion of 7.5%

Example 7

The following components were mixed in a conventional household blender:

250 ml urine (human)

500 ml water

250 gm. liquid pig manure

8 gm. enzymes mixture

Of this mixture, 800 ml was subjected to the proceedure of Example 5. Sample analysis indicated a conversion of 6%.

Example 8

The following components were mixed in a conventional household blender:

250 ml urine (human)

500 ml water

250 gm liquid pig manure

30 ml glycerine

8 gm. enzymes mixture

Of this mixture, 800 ml was subjected to the proceedure of Example 5. Sample analysis indicated a conversion of 9.7%.

Example 9

The following components were placed in a sealed container:

250 gm human faeces

1000 ml human urine

25 ml glycerine

The mix was held at 40°C for one month. 20 gm of enzymes mixture was then added, and the sample returned to the oven for a further seven days. Sample analysis indicated a conversion of 12%

Example 10

The mixture remaining from Example 3 was retreated. The material having aged somewhat was reheated under 500 psig of air pressure for 2 hours at 150°C. Sample analysis indicated a conversion of 57%.

Example 11

The following components were mixed in a conventional household blender:

150 gm old, wet, maggotty chicken manure

50 gm old, moldy, dry horse manure

250 ml old human urine

600 ml water

30 ml glycerine

8 ml sodium silicate

50 gm kaolin

10 gm enzyme mixture

This mixture was placed in a variable volume cylinder sealed with a piston, and provided with a pressure gauge. The free air space above the mixture was about one half the total space in the cylinder. After about 12 hours at ambient temperature (about $20^{o}C$) the cylinder piston was hydraulically pressurized to provide a pressure of 400 psig. and the cylinder heated to $150^{o}C$ for 2½ hours. After cooling, analysis of a sample indicated a conversion of 15%.

CLAIMS

1.      A process for converting biodegradable carbon-
containing materials into substances containing a significant proportion of methylene groups, which process
comprises subjecting the biodegradable carbon-containing
material  to bacterial action in a container under conditions
of temperature suitable for the bacterial flora present to
thrive, of pressure such that any evolved gasses are
retained in contact with the carbon-containing materials,
of volume such that the free space in the container is minimised,
and in the presence of at least sufficient water to maintain
the bacterial flora alive.

2.      A process according to claim 1 wherein the pressure ,
is the autogenous pressure developed in the
reaction.

3.      A process according to claim 1 including the additional
step of subjecting the material to a pressure of at least
400 psig and a temperature of at least 150$^{\circ}$C before removal
from the container.

4.      A process according to any one of the preceding
claims wherein a silica-containing material is added to
the carbonaceous material.

5.      A process according to any one of the preceding claims
wherein the silica containing material is an organic
silicone, sodium or potassium silicate, Fuller's earth,
bentonite, kaolin or kieselguhr.

6.      A process according to any one of the preceding
claims wherein the carbonaceous material is aged before    •

treatment to permit development of an adequate material bacterial flora.

7. A process according to any one of claims 1 to 5 wherein a bacterial flora is added to the carbonaceous material.

8. A process according to any one of the preceding claims wherein a bacterial stimulant or food is added to the carbonaceous material before treatment, said stimulant being an enzyme mixture and said food being glycerin or sugar.

9. A process according to any one of the preceding claims wherein the biodegradable carbon-containing material is sewage.

10. A process according to any one of claims 1 to 8 wherein the biodegradable carbon-containing material comprises urine, faeces or manure.

European Patent Office

**EUROPEAN SEARCH REPORT**

0045614

Application number

EP 81303445.1

| Category | Citation of document with indication, where appropriate, of relevant passages | Relevant to claim |
|---|---|---|
| | US - A - 4 041 182 (ERICKSON) <br> + Column 2, lines 19-37; column 8, lines 8-21 + <br> -- | 1,8-10 |
| P | GB - A - 2 039 943 (MOBIL OIL) (20-08-1980) <br> + Page 1, lines 5-10, 42-65; page 8, lines 61-65; page 9, lines 1-22 + <br> & BR-A-7 908 544 (22-07-1980) <br> -- | 1,3-5,8 |
| A | W. BAADER, E. DOHNE, M. BRENN-DÖRFER "Biogas in Therorie und Praxis" KTBL-Schrift 229, 1978 <br> KTBL-SCHRIFTEN-VERTRIEB IM LAND-WIRTSCHAFTSVERLAG GmbH, Münster-Hiltrup <br> + Page 12, lines 1-4, 10-21 + <br> ---- | 1 |

**DOCUMENTS CONSIDERED TO BE RELEVANT**

**CLASSIFICATION OF THE APPLICATION (Int Cl ³)**

C 02 F 3/28
C 02 F 11/04
A 01 C 3/00
A 01 C 3/02
C 12 P 5/00
C 12 P 5/02

**TECHNICAL FIELDS SEARCHED (Int. Cl. ³)**

C 02 F
A 01 C
C 12 P
C 10 G

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family.
corresponding document

| | | | |
|---|---|---|---|
| X | The present search report has been drawn up for all claims | | |

| Place of search | Date of completion of the search | Examiner |
|---|---|---|
| VIENNA | 15-10-1981 | WILFLINGER |

EPO Form 1503.1 06.78